# EUROPEAN PATENT APPLICATION

(11) **EP 1 961 422 A1**
(43) Date of publication of application: **27.08.2008**
(21) Application number: 06834663.4
(22) Date of filing: 14.12.2006
(51) Int. Cl.: A61K 31/522, A61K 47/12, A61K 47/22

(54) **EASILY ABSORBED ORAL PREPARATION CONTAINING XANTHINE DERIVATIVE**

(30) Priority: 14.12.2005 JP 2005360618
(71) Applicant: KYOWA HAKKO KOGYO CO., LTD., Tokyo 100-8185 (JP)
(72) Inventor: KIGOSHI, Makoto, Nagaizumi-cho, Sunto-gun Shizuoka 411-8731 (JP); KATO, Hideki, Chiyoda-ku, Tokyo 100-8185 (JP); AOKI, Noboru, Nagaizumi-cho, Sunto-gun Shizuoka 411-8731 (JP)
(74) Representative: Vossius & Partner
(86) International application number: PCT/JP2006/324909
(87) International publication number: WO 2007/069675

(57) **Abstract**

An object of the present invention is to provide an easily absorbable oral preparation containing a xanthine derivative or a pharmaceutically acceptable salt thereof and a fatty acid or a fatty acid derivative for the purpose of providing an easily absorbable oral preparation containing the xanthine derivative of which absorbability has been enhanced by dissolving the xanthine derivative that shows adenosine A₁ receptor antagonistic activity and has diuretic activity, kidney-protecting activity, bronchodilating activity, cerebral function-improving activity, anti-dementia activity or the like in a solvent.

## Description

### Technical Field

The present invention relates to an easily absorbable oral preparation of a xanthine derivative which shows adenosine A₁ receptor antagonistic activity and has diuretic activity, kidney-protecting activity, bronchodilating activity, cerebral function-improving activity, anti-dementia activity or the like.

### Background Art

Drugs that are hardly soluble in water and have high crystallinity generally have low bioavailability because of their low solubility and low dissolution rate in the gastrointestinal tract. In order to improve their low bioavailability, methods for finely grinding drug crystals, for transforming them into amorphous substances or for dissolving them in a solvent have heretofore been examined.

Finely grinding hardly soluble drugs or transforming them into amorphous substances involves a drawback that it is necessary to apply a strong pressure to them from the outside or heat them. Further, the methods of finely grinding drugs or of transforming them into amorphous substances involve a drawback that variation between lots is caused or drugs become unstable during the operation. In this respect, the methods of dissolving drugs have advantages of only requiring simple operation and making it less likely to cause variation between lots.

Solvents that can be used for oral preparations are selected from the viewpoint of safety. Examples of such solvents include ethanol, acetic acid, polysorbate, polyethylene glycol, sorbitan fatty acid esters and polyoxyethylene hydrogenated castor oil, and an example of solvents highly capable of dissolving hardly soluble drugs is polyethylene glycol. However, drugs that are hardly soluble in water and have high crystallinity are frequently hardly soluble in solvents and sometimes hardly soluble even in polyethylene glycol that is known to have a high solvent power. It has been considered to be difficult to improve the absorbability of such drugs by a method of dissolving them in solvents.

On the other hand, xanthine derivatives represented by formula (I): 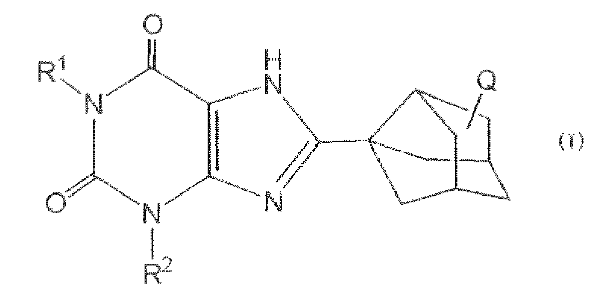 (wherein R¹ and R² are the same or different and represent substituted or unsubstituted lower alkyl, and Q represents hydrogen or hydroxy) or pharmaceutically acceptable salts thereof are considered to be useful as a pharmaceutical because they show adenosine A₁ receptor antagonistic activity and have diuretic activity, kidney-protecting activity, bronchodilating activity, cerebral function-improving activity, anti-dementia activity and the like. However, they are hardly soluble in water and also in polyethylene glycol.

As methods to improve absorbability of compounds (I), those described in patent document Nos. 1 and 2 have so far been known. However, it has not been known to improve their absorbability by a method of dissolving them in a solvent.
Patent document No. 1 : WO97/04782 pamphlet
Patent document No. 2 : WO02/43704 pamphlet

### Disclosure of the Invention

### Problems to be Solved by the Invention

An object of the present invention is to provide an easily absorbable oral preparation containing a xanthine derivative of which absorbability has been enhanced by dissolving the xanthine derivative that shows adenosine A₁ receptor antagonistic activity and has diuretic activity, kidney-protecting activity, bronchodilating activity, cerebral function-improving activity, anti-dementia activity or the like, in a solvent.

### Means for Solving the Problems

The present invention relates to the following (1) to (13).
(1) An easily absorbable oral preparation which comprises a xanthine derivative represented by formula (I): 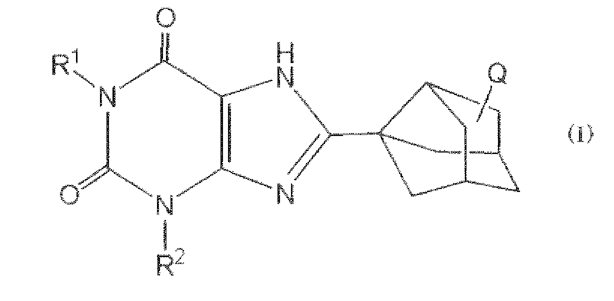 (wherein R¹ and R² are the same or different and represent substituted or unsubstituted lower alkyl, and Q represents hydrogen or hydroxy) or a pharmaceutically acceptable salt thereof and a fatty acid or a fatty acid derivative.
(2) The easily absorbable oral preparation according to the above (1), wherein the fatty acid or the fatty acid derivative is acetic acid, propionic acid, a saturated or unsaturated fatty acid having 4 to 22 carbon atoms, a saturated or unsaturated fatty acid in which the hydrogen atom of alkyl or alkenyl moiety having 3 to 21 carbon atoms is substituted by hydroxy or O(C₂H₄O)_{X}H (wherein X represents 0 to 100) or a derivative thereof.
(3) The easily absorbable oral preparation according to the above (1), wherein the fatty acid or the fatty acid derivative is a fatty acid derivative represented by formula (II): 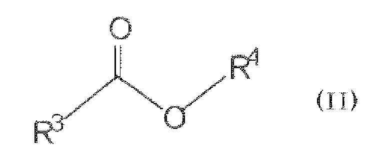 {wherein R³ represents methyl; ethyl; alkyl, alkenyl or alkadienyl having 3 to 21 carbon atoms; or alkyl, alkenyl or alkadienyl having 3 to 21 carbon atoms substituted by hydroxy or O(C₂H₄O)_{X}H (wherein X represents 0 to 100), and R⁴ represents hydrogen; methyl; ethyl; alkyl, alkenyl or alkadienyl having 3 to 21 carbon atoms; formula (III): 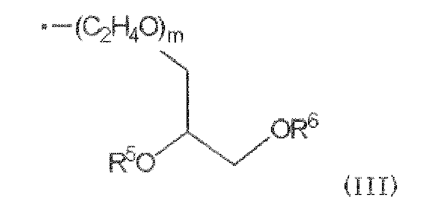 [wherein R⁵ and R⁶ are the same or different and represent hydrogen; methyl; ethyl; alkyl, alkenyl or alkadienyl having 3 to 21 carbon atoms; or (C₂H₄O)_{Y}COR⁷ (wherein R⁷ represents hydrogen; methyl; ethyl; alkyl, alkenyl or alkadienyl having 3 to 21 carbon atoms; or alkyl, alkenyl or alkadienyl having 3 to 21 carbon atoms substituted by hydroxy or O(C₂H₄O)_{Z}H (wherein Z represents 0 to 100), and Y represents 0 to 100), and m represents 0 to 100]; formula (IV): 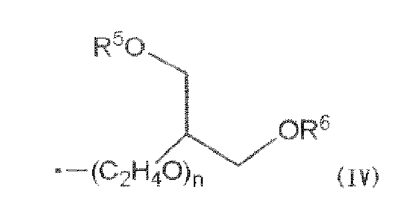 (wherein R⁵ and R⁶ have the same meanings as defined above respectively, and n represents 0 to 100); formula (V); 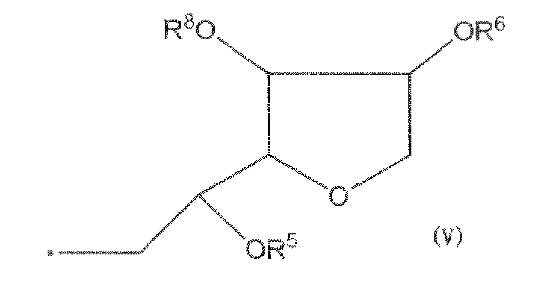 [wherein R⁵ and R⁶ have the same meanings as defined above respectively, and R⁸ represents hydrogen; methyl; ethyl; alkyl, alkenyl or alkadienyl having 3 to 21 carbon atoms; or (C₂H₄O)_{W}H (wherein W represents 0 to 100)]; or formula (VI): 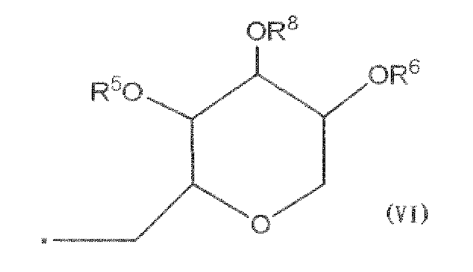 (wherein R⁵, R⁶ and R⁸ have the same meanings as defined above respectively)}.
(4) The easily absorbable oral preparation according to the above (3), wherein R³ is methyl; alkyl, alkenyl or alkadienyl having 11, 13, 15, 17 or 19 carbon atoms; or alkyl, alkenyl or alkadienyl having 11, 13, 15, 17 or 19 carbon atoms substituted by hydroxy or O(C₂H₄O)_{X}H (wherein X represents 0 to 100).
(5) The easily absorbable oral preparation according to the above (3), wherein R³ is methyl, cis-8-heptadecenyl, or cis-8-heptadecenyl or heptadecyl in which a carbon atom at position 11 is substituted by hydroxy or O(C₂H₄O)_{X}H (wherein X represents 0 to 100).
(6) The easily absorbable oral preparation according to any of the above (3) to (5), wherein R⁴ is formula (III), (IV), (V) or (VI), R⁵ and R⁶ are the same or different and are hydrogen or (C₂H_{4O})_{Y}COR⁷, at least one of R⁵ and R⁶ being (C₂H₄O)_{Y}COR⁷, and R⁷ is hydrogen; alkyl, alkenyl or alkadienyl having 15 to 21 carbon atoms; or alkyl, alkenyl or alkadienyl having 15 to 21 carbon atoms substituted by hydroxy or O(C₂H₄O)_{Z}H (wherein Z represents 0 to 100).
(7) The easily absorbable oral preparation according to any of the above (3) to (5), wherein R⁴ is formula (III), (IV) or (V), and R⁵ and R⁶ are the same or different and are hydrogen; or alkyl, alkenyl or alkadienyl having 15 to 21 carbon atoms.
(8) The easily absorbable oral preparation according to the above (1), wherein the fatty acid or the fatty acid derivative is a fatty acid or a fatty acid derivative which is available either as vinegar or olive oil.
(9) The easily absorbable oral preparation according to the above (1), wherein the fatty acid or the fatty acid derivative is one or more selected from acetic acid, octadecaenoic acid, hydroxyoctadecanoic acid, hydroxyoctadecaenoic acid and derivatives thereof.
(10) The easily absorbable oral preparation according to the above (1), wherein the fatty acid or the fatty acid derivative is one or more selected from acetic acid, oleic acid, monooleic acid sorbitan, trioleic acid sorbitan and polyoxyethylene sorbitan monooleate.
(11) The easily absorbable oral preparation according to the above (1), wherein the fatty acid or the fatty acid derivative is acetic acid or oleic acid.
(12) The easily absorbable oral preparation according to any of the above (1) to (11), which further comprises a fatty acid or a fatty acid derivative which is available as any of Wittepzol^{™} H5, Mittepzol^{™} H15, Wittepzol^{™} S51, Wittepzol^{™} S55, Gelucire^{™} and Suppocire^{™}.
(13) The easily absorbable oral preparation according to any of the above (1) to (12), which further comprises one or more selected from microcrystalline cellulose, hydrated silicon dioxide, light silicic anhydride, aluminum silicate, calcium silicate and magnesium methasilicate aluminate.

### Effect of the Invention

The present invention provides an easily absorbable oral preparation which comprises a xanthine derivative of which absorbability has been enhanced by a simple operation of dissolving the xanthine derivative that shows adenosine A₁ receptor antagonistic activity and has diuretic activity, kidney-protecting activity, bronchodilating activity, cerebral function-improving activity, anti-dementia activity or the like, in a solvent.

### Brief Description of the Drawings

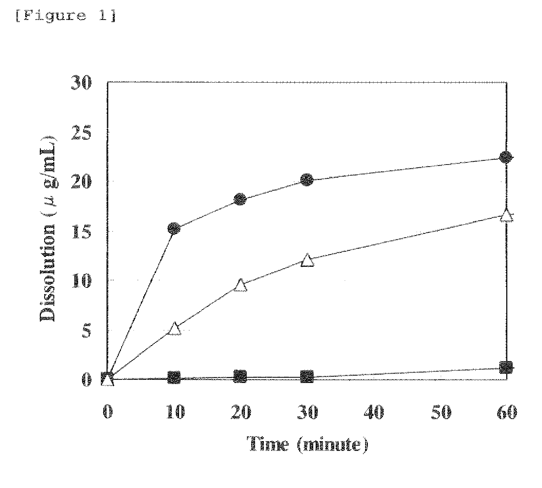

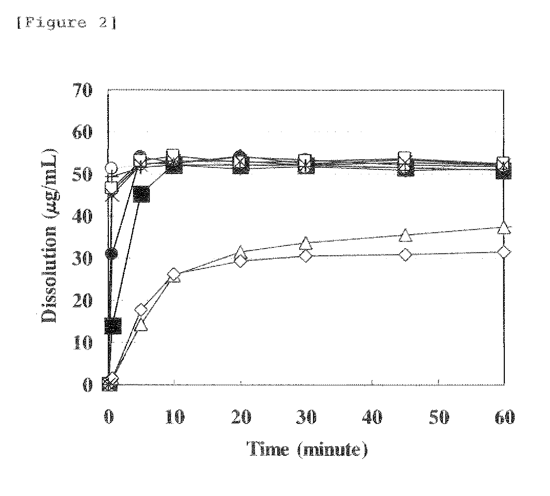

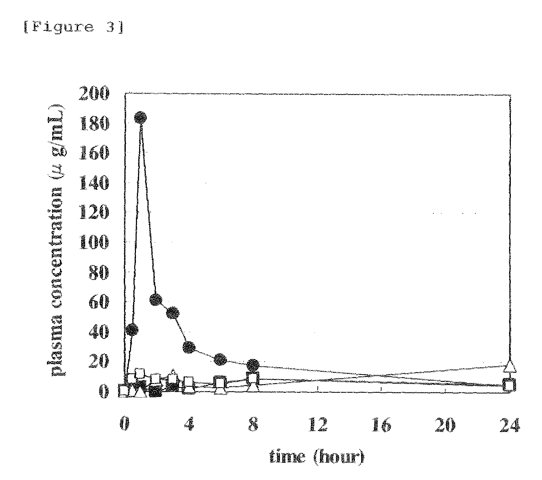

"Fig. 1" shows the result of an elution test of the preparation obtained in Example 1, a crystal of Compound (A) and the ground product of Comparative Example 1. A black circle shows the preparation of Example 1, a black square shows a crystal of Compound (A) and a white triangle shows the ground product of Comparative Example 1.
"Fig. 2" shows the results of elution tests of the preparations obtained in Examples 2 to 10. A black circle shows the preparation of Example 2, a black, square shows that of Example 3, a black rhombus shows that of Example 4, a white circle shows that of Example 5, a white square shows that of Example 6, a white triangle shows that of Example 7, a white rhombus shows that of Example 8, x-mark shows that of Example 9 and + shows that of Example 10.
"Fig 3" shows the result of an oral administration test of the preparation obtained in Example 1, a. crystal of Compound (A), the ground product of Comparative Example 1 and the preparation of Comparative Example 2 in beagle dogs. A black circle shows the preparation of Example 1, a black square shows a crystal of Compound (A), a white triangle shows the ground product of Comparative Example 1 and a white square shows the preparation of Comparative Example 2.

### Best Modes for Carrying Out the Invention

The xanthine derivatives of the present invention are xanthine derivatives represented by formula (I): 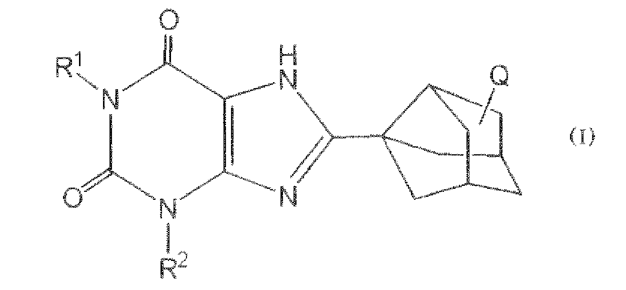 (wherein R¹ and R² are the same or different and represent substituted or unsubstituted lower alkyl, and Q represents hydrogen or hydroxy)[hereinafter referred to as "compounds (I)"]. In the definition of compounds (I), the lower alkyl includes straight chain or branched alkyl having 1 to 6 carbon atoms, for example, methyl, ethyl, propyl, isopropyl, butyl, isobutyl, sec-butyl, tert-butyl, pentyl, neopentyl, hexyl or the like. The substituent of the substituted lower alkyl includes, for example, hydroxy, acetyl or the like.

The pharmaceutically acceptable salts of compounds (I) include metal salts, ammonium salts, acid addition salts, organic amine addition salts and amino acid addition salts. Examples of metal salts include alkali metal salts such as lithium salt, sodium salt and potassium salt, alkaline earth metal salts such as magnesium salt and calcium salt, aluminum salt and zinc salt. Examples of ammonium salts include salts of ammonium and tetramethyl ammonium. Examples of acid addition salts include inorganic acid salts such as hydrochloride, sulfate, nitrate and phosphate and organic acid salts such as acetate, maleate, fumarate and citrate. Examples of organic amine addition salts include addition salts of morphorine, piperidine and the like, and examples of amino acid addition salts include addition salts of glycine, phenylalanine, aspartic acid, glutamic acid, lysine and the like.

Compounds (I) or pharmaceutically acceptable salts thereof can be produced according to the method described in Japanese Published Unexamined Patent Application No. 173889/91.

Specific examples of compounds (I) are shown in Table 1.

**Table 1**

| | | | |
|---|---|---|---|
| 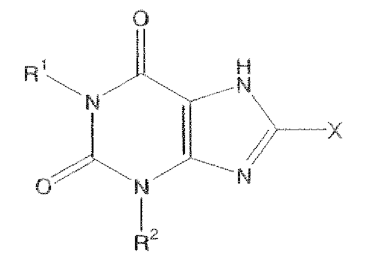 | | | |

| Compound Number | R¹ | R² | X |
|---|---|---|---|
| 1 | n-C₃H₇ | n-C₃H₇ | 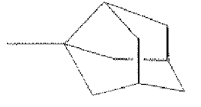 |
| 2 | n-C₃H₇ | n-C₃H₇ | 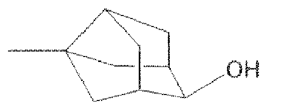 |
| 3 | n-C_{3H}7 | n-C₃H₇ | 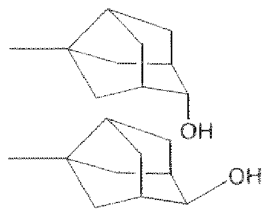 |
| 4 | 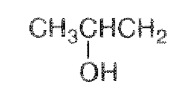 | n-C₃H₇ | |
| 5 | 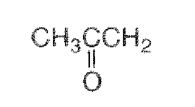 | n-C₃H₇ | 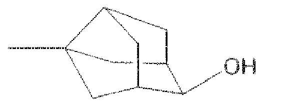 |

In the easily absorbable oral preparation of the present invention, the content of a compound (I) or a. pharmaceutically acceptable salt thereof in the easily absorbable oral preparation is preferably 1 to 50% by weight, more preferably 2 to 15% by weight and further preferably 2.5 to 10% by weight.

Fatty acids or fatty acid derivatives to be used in the present invention are not particularly limited so long as they can dissolve compounds (I) or pharmaceutically acceptable salts thereof. Examples of fatty acids include preferably acetic acid, propionic acid, saturated or unsaturated fatty acids having 4 to 22 carbon atoms and saturated or unsaturated fatty acids in which the hydrogen atom in the alkyl or alkenyl moiety having 3 to 21 carbon atoms is substituted by hydroxy or O(C₂H₄O)_{X}H (wherein X represents 0 to 100), more preferably acetic acid, saturated or unsaturated fatty acids having 12, 14, 16, 18 or 20 carbon atoms, and saturated or unsaturated fatty acids wherein the hydrogen atom in the alkyl or alkenyl moiety having 11, 13, 15, 17 or 19 carbon atoms is substituted by hydroxy or O(C₂H₄O)_{X}H (wherein X represents 0 to 100) and further preferably acetic acid, octadecaenoic acid, hydroxyoctadecanoic acid and hydroxyoctadecaenoic acid.

Examples of fatty acid derivatives are preferably fatty acid derivatives represented by formula (II): 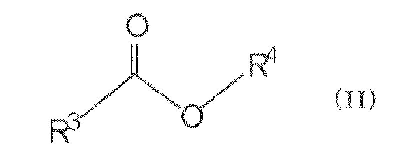 {wherein R³ represents methyl; ethyl; alkyl, alkenyl or alkadienyl having 3 to 21 carbon atoms; or alkyl, alkenyl or alkadienyl having 3 to 21 carbon atoms substituted by hydroxy or O(C₂H₄O)_{X}H (wherein X represents 0 to 100), and R⁴ represents hydrogen; methyl; ethyl; or alkyl, alkenyl or alkadienyl having 3 to 21 carbon atoms; formula (III): 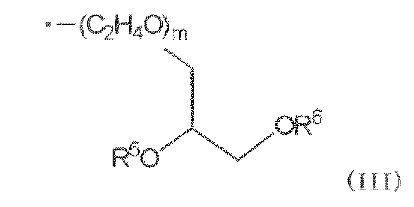 [wherein R⁵ and R⁶ are the same or different and represent hydrogen; methyl; ethyl; alkyl, alkenyl or alkadienyl having 3 to 21 carbon atoms; (C₂H₄O)_{Y}COR⁷ (wherein R⁷ represents hydrogen; methyl; ethyl; alkyl, alkenyl or alkadienyl having 3 to 21 carbon atoms; or alkyl, alkenyl or alkadienyl having 3 to 21 carbon atoms substituted by hydroxy or O(C₂H₄O)_{Z}H (wherein Z represents 0 to 100), and Y represents 0 to 100), and m represents 0 to 100]; formula (IV): 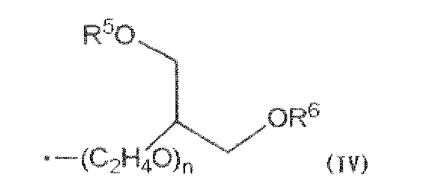 (wherein R⁵ and R⁶ have the same meanings as defined above respectively, and n represents 0 to 100); formula (V): 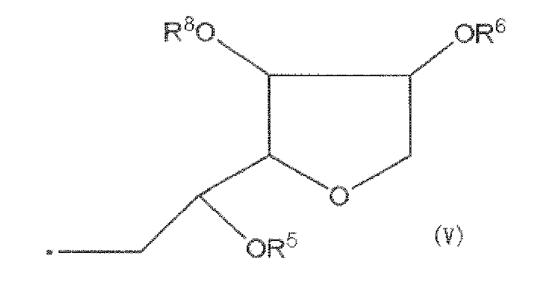 [wherein R⁵ and R⁶ have the same meanings as defined above respectively, and R⁸ represents hydrogen; methyl; ethyl; alkyl, alkenyl or alkadienyl having 3 to 21 carbon atoms; or (C₂H₄O)_{W}H (wherein W represents 0 to 100)]; or formula (VI): 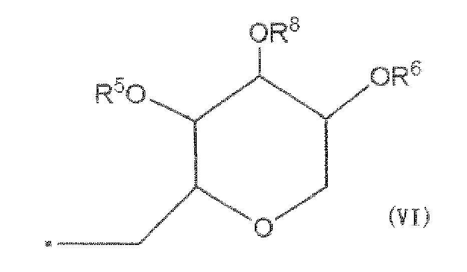 (wherein R⁵, R⁶ and R⁸ have the same meanings as defined above respectively)}.

R³ more preferably includes, for example, methyl; alkyl, alkenyl or alkadienyl having 11, 13, 15, 17 or 19 carbon atoms; or alkyl, alkenyl or alkadienyl having 11, 13, 15, 17 or 19 carbon atoms substituted by hydroxy or O(C₂H₄O)_{X}H (wherein X represents 0 to 100), and further preferably includes methyl, cis-8-heptadecenyl, or cis-8-heptadecenyl or heptadecyl in which a carbon atom at position 11 is substituted by hydroxy or O(C₂H₄O)_{X}H (wherein X represents 0 to 100).

R⁴ more preferably includes, for example, formula (III), (IV), (V) or (VI), in which case, it is preferred that R⁵ and R⁶ are the same or different and are hydrogen or (C₂H₄O)_{Y}COR⁷, at least one of R⁵ and R⁶ being (C₂H₄O)_{Y}COR⁷_{.} and R⁷ is hydrogen; alkyl, alkenyl or alkadienyl having 15 to 21 carbon atoms; or alkyl, alkenyl or alkadienyl having 15 to 21 carbon atoms substituted by hydroxy or O(C₂H₄O)_{Z}H (wherein Z represents 0 to 100). Further, when R⁴ is formula (III), (IV) or (V), it is also preferred that R⁵ and R⁶ are the same or different and are hydrogen, or alkyl, alkenyl or alkadienyl having 15 to 21 carbon atoms.

The most preferred examples of fatty acids and fatty acid derivatives are acetic acid, oleic acid, sorbitan monooleate, sorbitan trioleate and polyoxyethylene sorbitan monooleate. These solvents are allowed to be contained in oral preparations and have an advantage of requiring no removal.

Fatty acids and fatty acid derivatives can be obtained, for example, by extraction from animal fats and oils or vegetable fats and oils or by subjecting the obtained extracts to chemical decomposition, oxidization or reduction, or, otherwise, can be obtained by producing the derivatives according to known methods. It is also possible to obtain, for examples, castor oil, cacao seed oil, vinegar, olive oil or the like that contains the fatty acid or the fatty acid derivative of the present invention and use them as the fatty acid or the fatty acid derivative of the present invention as it is. In such case, vinegar and olive oil are more preferred.

In the easily absorbable oral preparation of the present invention, the ratio of a compound (I) or a pharmaceutically acceptable salt thereof to the fatty acid or the fatty acid derivative is preferably 1:1 to 1:99 (by weight), more preferably 1:3 to 1:50 (by weight) and further preferably 1:5 to 1:15 (by weight).

The easily absorbable oral preparation of the present invention can be prepared by dissolving a compound (I) or a pharmaceutically acceptable salt thereof in a fatty acid or a fatty acid derivative; and directly putting the resulting solution in a container to be prepared into an easily absorbable oral preparation as oral liquid or filling in capsules to be prepared into an easily absorbable oral preparation as capsules. A compound (I) or a pharmaceutically acceptable salt thereof may either be completely dissolved in a fatty acid or a fatty acid derivative or only partially dissolved with remainder being suspended therein. It is preferred that a compound (I) or a pharmaceutically acceptable salt thereof is completely dissolved in a fatty acid or a fatty acid derivative as variation between lots is not caused. However, it is likewise preferred even if a part thereof remains undissolved but is suspended, as variation between lots is suppressed due to high viscosity possessed by fatty acids and fatty acid derivatives.

A capsule form of an easily absorbable oral preparation is prepared by using a capsule filling equipment exclusively for soft capsules and seamless capsules. It is also possible to fill the solution in soft capsules and then seal the seam.

A compound (I) or a pharmaceutically acceptable salt thereof may be dissolved in a fatty acid or a fatty acid derivative using, for example, an ordinary agitator. An emulsifier and the like may also be used. It is also possible to promote dissolution by warming or reducing temperature, or pressurization or depressurization. In dissolving a pharmaceutical substance, the temperature and the time for the treatment vary depending upon the fatty acid or the fatty acid derivative used, but usually dissolution is carried out at room temperature to 100° C for several minutes to over 10 hours.

Further, a solution of a compound (I) or a pharmaceutically acceptable salt thereof in a fatty acid or a fatty acid derivative is mixed with an additive to form a suspension or a semi-solid/solution and prepared into an easily absorbable oral preparation in oral liquid preparation form or capsule form, or may also be prepared into an easily absorbable oral preparation in granule, fine granule or powder preparation form. When a compound (I) or a pharmaceutically acceptable salt thereof is dissolved in a fatty acid or a fatty acid derivative and is allowed to form a semi-solid/solution by adding an additive thereto, the concentration of the pharmaceutical substance is in the range of 1 to 30% by weight and preferably 2 to 20% by weight.

When an additive is mixed with a solution of a compound (I) or a pharmaceutically acceptable salt thereof in a fatty acid or a fatty acid derivative to form a suspension or a semi-solid/solution, the additive to be used includes, for example, polymers and self-emulsified emulsions. Examples of polymers include rubber compounds such as gum arabic; polysaccharides such as gelatin and agar; celluloses and cellulose derivatives such as microcrystalline cellulose, low-substituted hydroxypropylcellulose, carboxymethylcellulose, hydroxypropylmethylcellulose, hydroxypropylmethylcellulose phthalate, hydroxypropylmethylcellulose acetate succinate, carboxymethylethylcellulose, ethylcellulose and cellulose acetate phthalate; polyvinyl derivatives such as polyvinyl alcohol, polyvinyl pyrrolidone and polyvinylacetal diethylaminoacetate; aminoalkyl methacrylate copolymers such as aminoalkyl methacrylate copolymer E and aminoalkyl methacrylate copolymer RS; and methacrylic acid copolymers such as methacrylic acid copolymer L, methacrylic acid copolymer LD and methacrylic acid copolymer S. Among these polymers, methacrylic acid copolymer L (trade name: Eudragit L100, Eudragit L100-55; Rohm Pharma Co., Ltd.), hydroxypropylmethylcellulose phthalate (trade name: hydroxypropylmethylcellulose phthalate HP-55; Shin-Etsu Chemical Co., Ltd.), hydroxypropylmethylcellulose acetate succinate and carboxymethylethylcellulose are preferably used and methacrylic acid copolymer L (trade name: Eudragit L100) is more preferably used.

Examples of self-emulsified emulsions include products of wittepzol grade available as any of Wittepzol^{™} H5, Wittepzol^{™} H15, Wittepzol^{™} H32, Wittepzol^{™} S51, Wittepzol^{™} S55, Wittepzoll^{™} S58, Mittepzol^{™} W25 and Wittepzol^{™} W32, and preferred examples include products of wittepzol grade available as any of Wittepzol^{™} H5, Wittepzol^{™} H15, Wittepzol^{™} S51 and Wittepsol^{™} S55. Otherwise, examples of self-emulsified emulsions include products available as either of Gelucire^{™} and Suppocire^{™}, and preferred examples include products available as any of Gelucire^{™} 33/01, Gelucire^{™} 39/01, Gelucire^{™} 43/01 and Gelucire^{™} 44/14 or Suppocire^{™} Standard type, Suppocire^{™} N type and Suppocire^{™} P type.

When a solution of a compound (I) or a pharmaceutically acceptable salt thereof in a fatty acid or a fatty acid derivative is mixed with an additive to form a suspension or a semi-solid/solution, production is carried out using, for example, an agitator, an emulsifier, a vacuum emulsifier, a kneader, an agitation granulator and the like. In producing the easily absorbable oral preparation as capsules from the suspension or the semi-solid/solution, a filling equipment exclusively for a soft capsule and a seamless capsule is used. It is also possible to produce capsules by filling the suspension or the semi-solid/solution in hard capsules and sealing the seam. In producing the suspension or the semi-solid/solution, it is possible to raise the temperature or apply a pressure, and if the suspension or the semi-solid/solution is solidified after cooling or under atmospheric pressure, the easily absorbable oral preparation can be produced as granules, fine granules or powder.

It is also possible that a solution of a compound (I) or a pharmaceutically acceptable salt thereof in a fatty acid or a fatty acid derivative, or a suspension or a semi-solid/solution obtained by mixing a solution of a compound (I) or a pharmaceutically acceptable salt thereof in a fatty acid or a fatty acid derivative with an additive is further mixed with an additive, and the resulting mixture is allowed to be absorbed by or adsorbed on the solid additive to become solid and prepared into an easily absorbable oral preparation as granules, fine granules, powder, capsules, tablets or the like.

In case a solution of a compound (I) or a pharmaceutically acceptable salt thereof in a fatty acid or a fatty acid derivative, or a. suspension or a semi-solid/solution obtained by mixing a solution of a compound (I) or a pharmaceutically acceptable salt thereof in a fatty acid or a fatty acid derivative with an additive is further mixed with an additive and the resulting mixture is allowed to be absorbed by or absorbed on the solid additive to become solid, the additive to be further mixed includes excipients and the like that are usually used in the field of drug preparation, specifically, monosaccharides or oligosaccharides such as glucose, lactose, sucrose, maltose and trehalose; sugar alcohols such as mannitol, maltitol, maltol, lactitol, xylitol, sorbitol and erythritol; starches and derivatives thereof such as corn starch, potato starch, dextrin and pullulane; celluloses such as microcrystalline cellulose, etc.; amino acids such as arginine, asparagine, aspartic acid, citrulline, cysteine, glutamic acid, glutamine, glycine, histidine, homoserine, isoleucine, leucine, lysine, methionine, ornithine, phenylalanine, proline, serine, threonine, tryptophan, tyrosine and valine; and inorganic substances such as hydrated silicon dioxide, light silicic anhydride, aluminum silicate, synthetic hydrotalcite, titanium oxide, aluminum hydroxide, talc, calcium carbonate, calcium silicate, bentonite, magnesium methasilicate aluminate, calcium hydrogenphosphate and calcium hydrogenphosphate anhydride, and more preferably includes microcrystalline cellulose, hydrated silicon dioxide, light silicic anhydride, aluminum silicate, calcium silicate and magnesium methasilicate aluminate. In the easily absorbable oral preparation of the present invention, the ratio of the above-mentioned additive to be used for absorption or adsorption to make the mixture solid is preferably 1 to 90% by weight, more preferably 15 to 60% by weight and further preferably 20 to 40% by weight.

To achieve a solid form of a solution of a compound (I) or a pharmaceutically acceptable salt thereof in a fatty acid or a fatty acid derivative, or a suspension or a semi-solid/solution obtained by mixing a solution of a compound (I) or a pharmaceutically acceptable salt thereof in a fatty acid or a fatty acid derivative with an additive, which is further mixed with an additive and allowed to be absorbed by or adsorbed on the solid additive, an agitator, a kneader, an agitation granulator, a spray drying granulator and a vacuum drying granulator, for example, can be used. The resulting solid matter can be prepared into an easily absorbable oral preparation such as powder and granules, It is possible that the solid matter is prepared into an easily absorbable oral preparation such as powder, granules, capsules and tablets through further processing steps of drug preparation according to conventional methods, for example, mixing step, granulation processing step, tableting step, encapsulating step and coating step.

The easily absorbable oral preparation of the present invention may contain, in addition to a compound (I) or a pharmaceutically acceptable salt thereof, a fatty acid or a fatty acid derivative, the above-mentioned additive to form a semi-solid/solution and the above-mentioned additive to be used for absorption or adsorption to make the mixture solid, other active ingredients and/or additive (for example, excipients, disintegrants, binding agents, lubricants, light blocking agents or coloring agents, taste corrigents and surfactants). These additives can be added by an appropriate method with respect to each step. Examples of excipiets include monosaccharides or oligosaccharides (for example, lactose, sucrose and maltose), sugar alcohols (for example, mannitol, maltitol and erythritol), starches (for example, corn starch, rice starch and wheat starch), celluloses (for example, crystalline cellulose and powder cellulose), cellulose derivatives (for example, methylcellulose, carboxypropylcellulose, hydroxypropylmethylcellulose, croscarmellose sodium, low-substituted hydroxypropylcellulose, carboxymethylcellulose and carboxymethylcellulose calcium), talc and light silicic anhydride, more preferably one or more substances selected from monosaccharides or oligosaccharides, starches, celluloses and cellulose derivatives (specifically, sucrose and lactose among monosaccharides or oligosaccharides, corn starch among starches, crystalline cellulose among celluloses, croscarmellose sodium, low-substituted hydroxypropylcellulose, carboxymethyl cellulose or cellulose calcium among cellulose derivatives), and further preferably one or more substances selected from monosaccharides or oligosaccharides, starches and celluloses (specifically, lactose, sucrose, corn starch and crystalline cellulose). Examples of disintegrants include celluloses (for example, crystalline cellulose and powder cellulose), cellulose derivatives (for example, croscarmellose sodium, low-substituted hydroxypropylcellulose and carmellose calcium), starches (for example, corn starch, α -starch, partial α - starch and hydroxypropyl starch), crospovidone and bentonite. Examples of binding agents include cellulose derivatives (for example, methylcellulose, carboxymethylcellulose, carboxypropylcellulose, hydroxypropylcellulose and hydroxypropylmethylcellulose), celluloses (for example, crystalline cellulose), starches (for example, α -starch), polyvinyl alcohol, polyvinylpyrrolidone, pullulane, dextrin, gum arabic and gelatin. Examples of lubricants include magnesium stearate, calcium stearate, hydrogenated oils, sucrose fatty acid esters and polyethylene glycols. Examples of light blocking agents or coloring agents include titanium oxide, iron oxides, zinc oxide, colcothar, carbon black, medicinal carbon, barium sulfate, Food Yellow No. 4 Aluminum Lake, Food Red No. 2, Food Red No. 3, Food Red No. 102, copper chlorofin and silicon oxide, preferably titanium oxide, iron oxides (specifically, yellow iron sesquioxide, iron sesquioxide, yellow iron oxide and black iron oxide), zinc oxide, silicon oxide, various food colors and copper chlorofin sodium, further preferably titanium oxide and iron oxides (specifically, yellow iron sesquioxide and iron sesquioxide) and most preferably titanium oxide. Examples of taste corrigents include sucrose, saccharin, aspartame, mannitol, maltitol, erythritol, dextran, lemon flavors, menthol and citric acid. Examples of surfactants include sodium lauryl sulfate, polysorbate 80 and sucrose fatty acid esters.

When an automatic encapsulating or a tableting machine is used, it is preferred to mix 0.5 to 5% by weight of a lubricant before the encapsulating or tableting step to prevent adhesion of powder onto the piston or punch.

The present invention is illustrated more in detail below on the basis of examples and test examples. However, the present invention is not restricted to these examples and test examples. In the examples and test examples, Compound 1 in Table 1 [hereinafter referred to as "Compound (A)"] was used among compounds (I). However, the easily absorbable oral preparation containing a xanthine derivative with enhanced absorbability can be obtained using other compounds (I) and the present invention is not restricted to Compound (A).

### Test Example 1

Compound (A) was mixed with the various solvents shown in Table 2, respectively, and the mixture was stirred for 1 hour or 2 hours under warming at 50°C (room temperature with respect to methanol, ethanol, acetonitrile and acetic acid). Compound (A) was then quantitatively determined according to the method for quantitative determination described below and the concentration (mg/g) thereof in the various solvents was assayed. The result is shown in Table 2.

### Method for quantitative determination of Compound (A)

High performance liquid chromatography
Column: Inertsil ODS-2; 4.6 x 150mm; GL Sciences Inc.
Column temperature: Room temperature
Mobile phase: 65% acetonitrile
Detection method: Ultraviolet spectrophotometry (wave length: 280nm)

**Table 2**

| Solvent | Solubility (compound (A)/ solvent = mg/g) |
|---|---|
| Methanol (Kanto Kagaku) | 12 |
| Ethanol (Kanto Kagaku) | 18 |
| Acetonitrile (Kanto Kagaku) | 1.4 |
| Polyethylene glycol 400 (NOF Corporation) | < 10 |
| Acetic Acid (Kanto Kagaku) | 100 < |
| Polyoxyethylene monooleate (Tween 80; Tokyo Chemical Industry) | 16.0 |
| Sorbitan monolaurate (Span 20; Tokyo Chemical Industry) | 10.2 |
| Sorbitan monooleate (Span 80; Tokyo Chemical Industry) | 17.8 |
| Sorbitan trioleate (Span 85; Tokyo Chemical Industry) | 38.2 |
| Soybean oil (Kanto Kagaku) | 13.8 |
| Oleic acid (NOF Corporation) | 100 < |
| Polyoxyethylene hydrogenated castor oil (HCO-40; Nikko Chemicals) | 92.3 |

### Example 1

Compound (A) was dissolved in oleic acid (NOF Corporation) and the resulting solution was mixed with calcium silicate (Fluorite; Eisai) at a weight ratio of 4:1 to obtain granules. This preparation was formulated to contain 8% by weight of Compound (A), 73.6 % by weight of oleic acid and 18.4% by weight of calcium silicate.

### Example 2

Compound (A)(0.06 g) was dissolved in 1.98 g of oleic acid to obtain an oral liquid preparation.

### Example 3

Compound (A)(0.06 g) and 0.18 g of methacrylate copolymer L (Rohm Pharma) were dissolved in 1.76 g of oleic acid to obtain an oral liquid preparation.

### Example 4

Compound (A)(0.1 g) was dissolved in 0.96 g of oleic acid to obtain an oral liquid preparation.

### Example 5

Compound (A)(0.6 g) was dissolved in 5.4 g of oleic acid (hereinafter referred to as "liquid A") and 0.9 g of liquid A and 2.1 g of microcrystalline cellulose (Avicel PH-101; Asahi Kasei) were stirred in a mortar to obtain a granule preparation.

### Example 6

Compound. (A)(0.6 g) and 1.8 g of methacrylate copolymer L were dissolved in 5.4 g of oleic acid (hereinafter referred to as "liquid B") and 1.1 g of liquid B and 1.8 g of microcrystalline cellulose were stirred in a mortar to obtain a granule preparation.

### Example 7

Liquid A (0.9 g) and 0.9 g of magnesium methasilicate aluminate (Neusilin US2; Fuji Chemical Industry) were stirred and then mixed with 1.2 g of lactose to obtain a granule preparation.

### Example 8

Liquid B (1.2 g) and 0.9 g of magnesium methasilicate aluminate were stirred and then mixed with 0.9 g of lactose to obtain a granule preparation.

### Example 9

Liquid A (1.0 g) and 0.9 g of light silicic anhydride (Adsolider 101; Freund) were stirred and then mixed with 1.2 g of lactose (DMV) to obtain a granule preparation.

### Example 10

Liquid B (1.2 g) and 0.9 g of light silicic anhydride were stirred and then mixed with 0.9 g of lactose to obtain a granule preparation.

### Comparative Example 1

Compound (A) was put in a hammer (laboratory mill) grinder to obtain a ground product having mean volume diameter of about 23.9 µm.

### Comparative Example 2

A ground product of Compound (A), lactose, corn starch (Nihon Shokuhin Kako) and crospovidone (Polyplasdone XL-10; ISP Japan) were subjected to fluidized-bed granulation using a liquid prepared by dissolving polyvinyl alcohol (The Nippon Synthetic Chemical Industry Co., Ltd.) in distilled water as a liquid binding agent and the resulting granulated product was mixed with magnesium stearate (Sakai Chemical) to prepare tablets. One tablet contained 39.0 mg of the ground product of Compound (A).

### Test Example 2

The preparation obtained in Example 1 (0.4 g) was put in 800 ml of an aqueous solution containing 0.3% by weight of sodium lauryl sulfate (Nikko Chemicals) at 37°C, and an elution test was carried out by a puddle method under the condition of 100 min⁻¹. Elution tests were also carried out with respect to 51 mg of a crystal of Compound (A) and 51 mg of the ground product of Compound (A) obtained in Comparative Example 1, respectively, in the same manner using 900 ml of the elution liquid. The results are shown in Fig. 1. As shown in Fig. 1, the preparation obtained in Example 1 had higher elution rate than the crystal of Compound (A) and the ground product of Compound (A) obtained in Comparative Example 1.

### Method for quantitative determination of Compound (A) in the elution tests

High performance liquid chromatography
Column: Inertsil C8; 4.6 x 250mm; GL Sciences Inc.
Column temperature: Room temperature
Mobile phase: 0.02 mol/L phosphate buffer (pH 3.0):
acetonitrile = 450 mL:550 mL
Detection method: Ultraviolet spectrophotometry (wave length: 280nm)

### Test Example 3

Elution tests were carried out with respect to each 1.7 g of the preparations obtained in Examples 2 to 10 (0.5 g only for the preparation of Example 4), respectively, in the same manner using 900 ml of the elution liquid of the above Test Example 1. The results are shown in Fig. 2.

### Test Example 4

The preparation obtained in Example 1 was filled in capsules (Elanco Japan) to obtain a capsule preparation containing 39 mg of Compound (A) per 1 capsule. To 5 male beagle dogs, capsules equivalent to 78 mg as Compound (A) (6 mg/kg body weight) were orally administered together with 20 ml of water and absorbability after oral administration was evaluated. For comparison, a capsule preparation obtained by filling 78 mg of a crystal of Compound (A) in hard gelatin capsules, a capsule preparation obtained by filling 78 mg of the ground product of Comparative Example 1 in hard gelatin capsules and the tablets obtained in Comparative Example 2 were likewise administered to beagle dogs, respectively (Crystal of Compound (A): 2 doge; Comparative Example 1: 2 dogs; Comparative Example 2: 5 dogs). Of the result of this comparative experiment on absorption, transition of the concentration of these preparations in plasma is shown in Fig. 3 and pharmacokinetic parameters are shown in Table 3.

**Table 3**

| Preparation administered | Area under the plasma concentration-time curve (0-24.ng h/ml) | Maximum concentration in plasma Cmax (ng) |
|---|---|---|
| Example 1 | 545.6±352.5 | 185.5 |
| Crystal of compound (A) | 139.7 | 9.0 |
| Comparative Example 1 | 217.4 | 22.0 |
| Comparative Example 2 | 165.4±107.0 | 14.9 |

As a result of the comparative experiment on absorption, the preparation obtained in Example 1 possessed excellent absorbability superior to those of the crystal of Compound (A), the ground product of Comparative Example 1 and the preparation of Comparative Example 2, more than twofold in terms of the area under the plasma concentration-time curve and more than eightfold in terms of maximum concentration in plasma.

Further, as the preparations obtained in Examples 2 to 10 showed higher elution rate as a result of the elusion test of Test Example 2 than that of the preparation (of Example 1) that showed excellent absorbability in this comparative experiment on absorption, they were considered to likewise show high oral absorbability.

### Example 11

Oleic acid (180 kg) is put in a. 300 L-solution tank in which 20 kg of Compound (A) is further put and the mixture is allowed to dissolve by stirring at room temperature for 30 minutes. The solution is filled in a film consisting of gelatin, glycerin and the like at a liquid volume of 100 mg ± 3% using a soft capsule production line to obtain a soft capsule preparation with each capsule containing 10 mg of Compound (A).

### Example 12

Oleic acid (27 kg) is put in a 30 L-solution tank in which 3 kg of Compound (A) is further put and the mixture is allowed to dissolve by stirring at room temperature for 30 minutes. Microcrystalline cellulose (69 kg) is put in an agitation granulator and the solution obtained above is dropped thereto and mixed with stirring. To the mixture is further added 1 kg of magnesium stearate and mixed for one minute. The granules produced by this step are encapsulated under the conditions of capsule size No. 1 and the mass of the contents of 333 ± 10 mg using a capsule filling machine to obtain a. hard capsule preparation with one capsule containing 10 mg of Compound (A).

### Example 13

Oleic acid (27 kg) is put in a 30 L-solution tank in which 3 kg of Compound (A) is put and the mixture is allowed to dissolve by stirring at room temperature for 30 minutes. Microcrystalline cellulose (69 kg) is put in an agitation granulator and the solution obtained above is dropped thereto and mixed with stirring. To the mixture is further added 1 kg of magnesium stearate and mixed for one minute. The granules produced by this step are subjected to tableting under the condition of the mass of the contents of 333 ± 10 mg using a tableting machine to obtain tablets each containing 10 mg of Compound (A).

### Industrial Applicability

According to the present invention, an easily absorbable oral preparation containing a xanthine derivative of which absorbability has been enhanced by a simple operation of dissolving the xanthine derivative that shows adenosine A₁ receptor antagonistic activity and has diuretic activity, kidney-protecting activity, bronchodilating activity, cerebral function-improving activity, anti-dementia activity or the like in a solvent can be provided.

## Claims

1. An easily absorbable oral preparation which comprises a xanthine derivative represented by formula (I): 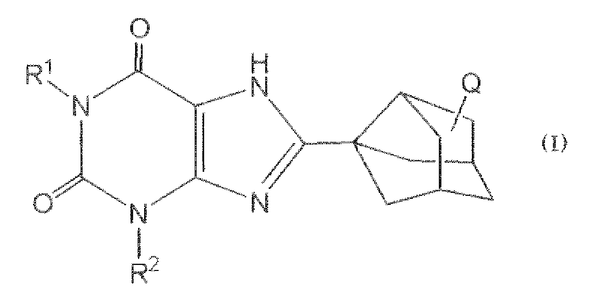 (wherein R¹ and R² are the same or different and represent substituted or unsubstituted lower alkyl, and Q represents hydrogen or hydroxy) or a pharmaceutically acceptable salt thereof and a fatty acid or a fatty acid derivative.

2. The easily absorbable oral preparation according to claim 1, wherein the fatty acid or the fatty acid derivative is acetic acid, propionic acid, a saturated or unsaturated fatty acid having 4 to 22 carbon atoms, a saturated or unsaturated fatty acid in which the hydrogen atom of alkyl or alkenyl moiety having 3 to 21 carbon atoms is substituted by hydroxy or O(C₂H₄O)_{X}H (wherein X represents 0 to 100) or a derivative thereof.

3. The easily absorbable oral preparation according to claim 1, wherein the fatty acid or the fatty acid derivative is a fatty acid derivative represented by formula (II): 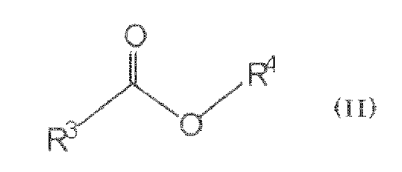 {wherein R³ represents methyl: ethyl; alkyl, alkenyl or alkadienyl having 3 to 21 carbon atoms; or alkyl, alkenyl or alkadienyl having 3 to 21 carbon atoms substituted by hydroxy or O(C₂H₄O)_{X}H (wherein X represents 0 to 100), and R⁴ represents hydrogen; methyl, ethyl; alkyl, alkenyl or alkadienyl having 3 to 21 carbon atoms; formula (III): 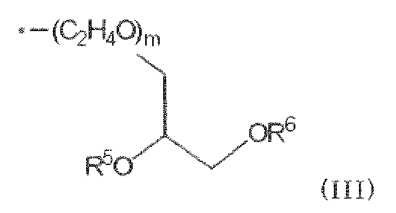 [wherein R⁵ and R⁶ are the same or different and represent hydrogen; methyl; ethyl; alkyl, alkenyl or alkadienyl having 3 to 21 carbon atoms; or (C₂H₄O)_{Y}COR₇ (wherein R⁷ represents hydrogen; methyl; ethyl; alkyl, alkenyl or alkadienyl having 3 to 21 carbon atoms; or alkyl, alkenyl or alkadienyl having 3 to 21 carbon atoms substituted by hydroxy or O(C₂H₄O)_{Z}H (wherein Z represents 0 to 100), and Y represents 0 to 100), and m represents 0 to 1001; formula (IV): 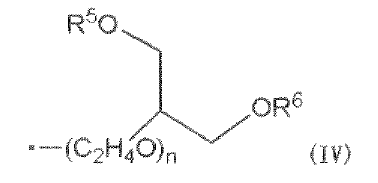 (wherein R⁵ and R⁶ have the same meanings as defined above respectively, and n represents 0 to 100}; formula (V): 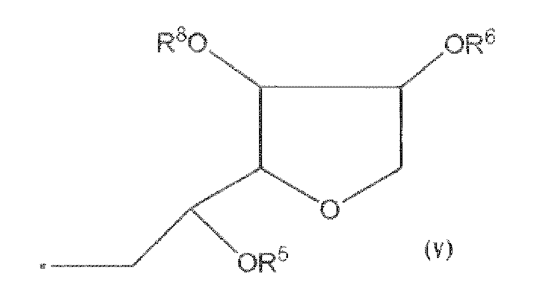 [wherein R⁵ and R⁶ have the same meanings as defined above respectively, and R⁸ represents hydrogen; methyl; ethyl; alkyl, alkenyl or alkadienyl having 3 to 21 carbon atoms; or (C₂H₄O)_{W}H (wherein W represents 0 to 100)]; or formula (VI): 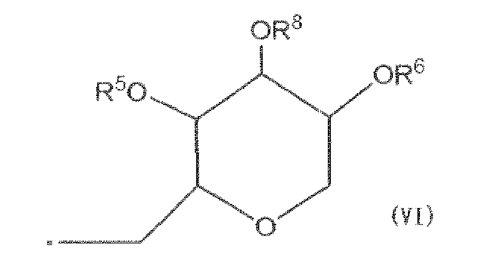 (wherein R⁵, R⁶ and H⁸ have the same meanings as defined above respectively)}.

4. The easily absorbable oral preparation according to claim 3, wherein R³ is methyl; alkyl, alkenyl or alkadienyl having 11, 13, 15, 17 or 19 carbon atoms; or alkyl, alkenyl or alkadienyl having 11, 13, 15, 17 or 19 carbon atoms substituted by hydroxy or O(C₂H₄O)_{X}H (wherein X represents 0 to 100).

5. The easily absorbable oral preparation according to claim 3, wherein R³ is methyl, cis-8-heptadecenyl, or cis-8-heptadecenyl or heptadecyl in which a carbon atom at position 11 is substituted by hydroxy or O(C₂H₄O)_{X}H (wherein X represents 0 to 100).

6. The easily absorbable oral preparation according to any of claims 3 to 5, wherein R⁴ is formula (III), (IV), (V) or (VI), R⁵ and R⁶ are the same or different and are hydrogen or (C₂H₄O)_{Y}COR⁷, at least one of R⁵ and R⁶ being (C₂H₄O)_{Y}COR⁷, and R⁷ is hydrogen; alkyl, alkenyl or alkadienyl having 15 to 21 carbon atoms; or alkyl, alkenyl or alkadienyl having 15 to 21 carbon atoms substituted by hydroxy or O(C₂H₄O)_{Z}H (wherein Z represents 0 to 100).

7. The easily absorbable oral preparation according to any of claims 3 to 5, wherein R⁴ is formula (III), (IV) or (V), and R⁵ and R⁶ are the same or different and are hydrogen; or alkyl, alkenyl or alkadienyl having 15 to 21 carbon atoms.

8. The easily absorbable oral preparation according to claim 1, wherein the fatty acid or the fatty acid derivative is a fatty acid or a fatty acid derivative which is available either as vinegar or olive oil.

9. The easily absorbable oral preparation according to claim 1, wherein the fatty acid or the fatty acid derivative is one or more selected from acetic acid, octadecaenoic acid, hydroxyoctadecanoic acid, hydroxyoctadecaenoic acid and derivatives thereof.

10. The easily absorbable oral preparation according to claim 1, wherein the fatty acid or the fatty acid derivative is one or more selected from acetic acid, oleic acid, monooleic acid sorbitan, trioleic acid sorbitan and polyoxyethylene sorbitan monooleate.

11. The easily absorbable oral preparation according to claim 1, wherein the fatty acid or the fatty acid derivative is acetic acid or oleic acid.

12. The easily absorbable oral preparation according to any of claims 1 to 11, which further comprises a fatty acid or a fatty acid derivative which is available as any of Wittepzol^{™} H5, Wittepzol^{™} H15, Wittepzol^{™} S51, wittepzol^{™} S55, Gelucire^{™} and Suppocire^{™}.

13. The easily absorbable oral preparation according to any of claims 1 to 12, which further comprises one or more selected from microcrystalline cellulose, hydrated silicon dioxide, light silicic anhydride, aluminum silicate, calcium silicate and magnesium methasilicate aluminate.
